(19) Europäisches Patentamt / European Patent Office / Office européen des brevets

(11) **EP 4 324 495 A1**

(12) **EUROPEAN PATENT APPLICATION**
published in accordance with Art. 153(4) EPC

(43) Date of publication:
**21.02.2024 Bulletin 2024/08**

(21) Application number: **22788215.6**

(22) Date of filing: **15.04.2022**

(51) International Patent Classification (IPC):
**A61M 1/18** (2006.01)   **A61M 1/36** (2006.01)

(52) Cooperative Patent Classification (CPC):
**A61M 1/36; B01D 63/02**

(86) International application number:
**PCT/JP2022/017891**

(87) International publication number:
**WO 2022/220292 (20.10.2022 Gazette 2022/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.04.2021   JP 2021068934**

(71) Applicant: **Toray Industries, Inc.**
**Tokyo 103-8666 (JP)**

(72) Inventors:
- **YAMASHITA, Kyohei**
  **Otsu-shi, Shiga 520-8558 (JP)**
- **TAKAHASHI, Hiroshi**
  **Otsu-shi, Shiga 520-8558 (JP)**

(74) Representative: **Kador & Partner Part mbB**
**Corneliusstraße 15**
**80469 München (DE)**

(54) **BLOOD TREATMENT MATERIAL**

(57)   An object of the present invention is to provide a blood treatment material having both the water permeability to allow removal of water from the blood and the ability to highly efficiently adsorb and remove, for example, inflammatory cells and inflammatory cytokines. As a means for achieving the object, the present invention provides a blood treatment material including a hollow fiber membrane having a surface modified with a ligand containing an amino group(s), wherein the arithmetic mean roughness (RaA) of the blood-contacting surface of the hollow fiber membrane in the radial direction calculated by using a laser microscope is from 0.10 $\mu$m to 0.80 $\mu$m.

Fig. 1

EP 4 324 495 A1

**Description**

Technical Field

The present invention relates to a blood treatment material.

Background Art

Hollow fiber membranes are cylindrical semi-permeable membranes that are generally known to have a function to allow for selective permeation of substances through micropores on the surface.

Therefore, hollow fiber membranes are used in liquid separation processes for various applications, including medical applications such as hemodialysis and blood filtration. For hemodialysis application, a method is known in which the blood is caused to flow through the inside of a hollow fiber membrane to allow water and small molecules such as uremic toxins in the blood to permeate and diffuse outside the hollow fiber membrane for removal.

For example, Patent Literature 1 discloses that a hollow fiber membrane made of a copolymer of acrylonitrile and sodium methallyl sulfonate with an immobilized ligand such as an IgG antibody can be used to adsorb and remove proteins such as IL-6.

Patent Literature 2 discloses that the positive charge induced on the surface of a hollow fiber membrane made of a copolymer of acrylonitrile and sodium methallyl sulfonate by introducing polyethyleneimine into the surface can inhibit the contact phase activation during extracorporeal treatment of, for example, the blood and prevent the generation of kallikrein in the blood, leading to the reduction of anaphylactoid reaction.

Patent Literature 3 discloses that a hollow fiber membrane made of poly(ether-sulfone) and containing ion-exchange particles can be used to adsorb and remove DNA.

Recently, it has become known that for the treatment of acute renal failure patients complicated by septic shock or chronic renal failure patients with unstable circulatory dynamics, the removal of small molecules and proteins in the blood by blood purification therapy is not sufficient and the additional removal of inflammatory cells, such as activated leukocytes, from the blood is desired.

As an example of a material for removing inflammatory cells such as activated leukocytes, a material comprising a sea-island composite fiber having a surface modified with a ligand containing an amino group(s) is known (Patent Literature 4).

Citation List

Patent Literature

Patent Literature 1: JP 4224621 B
Patent Literature 2: JP 4579916 B
Patent Literature 3: JP 6378090 B
Patent Literature 4: JP 5298719 B

Summary of Invention

Technical Problem

However, Patent Literature 1 discloses the ability of the hollow fiber membrane with an IgG antibody-immobilized surface to adsorb IL-6, but does not describe the ability to adsorb activated leukocytes.

Patent Literature 2 disclose that the hollow fiber membrane is for delaying the contact phase activation while preventing endotoxins removed from the blood during extracorporeal circulation from returning to the blood by the reverse filtration, but does not describe adsorption and removal of inflammatory cells such as activated leukocytes.

The hollow fiber membrane described in Patent Literature 3 physically contains hydrophilic particles and therefore has a risk of releasing the hydrophilic particles into the blood when used for extracorporeal circulation.

As described above, hollow fiber membrane blood treatment materials have been developed for the purpose of diffusing and removing water and proteins such as inflammatory cytokines in the blood, but not for adsorbing inflammatory cells such as activated leukocytes. Therefore, a hollow fiber membrane is expected to be developed that can not only remove water and the like in the blood, but also adsorb and remove pathogenic factors and substances, such as inflammatory cells and inflammatory cytokines.

Thus, an object of the present invention is to provide a blood treatment material having both the water permeability to allow removal of water from the blood and the ability to highly efficiently adsorb and remove, for example, inflammatory cells and inflammatory cytokines.

Solution to Problem

The inventors intensely studied to solve the above problems and consequently found that both water permeability and the ability to highly efficiently adsorb and remove, for example, inflammatory cells and inflammatory cytokines can be achieved in a hollow fiber membrane by making the blood-contacting surface of the membrane rough.

That is, the present invention includes (1) to (7) below.

(1) A blood treatment material comprising a hollow fiber membrane having a surface modified with a ligand containing an amino group(s), wherein the arithmetic mean roughness (RaA) of the blood-contacting surface of the hollow fiber membrane in the radial direction calculated by using a laser microscope is from 0.10 $\mu$m to 0.80 $\mu$m.

(2) The blood treatment material of (1), wherein the arithmetic mean roughness (RaA) is from 0.30 $\mu$m to 0.60 $\mu$m.

(3) The blood treatment material of (1) or (2), wherein the blood-contacting surface is the inner surface of the hollow fiber membrane.

(4) The blood treatment material of any of (1) to (3), wherein the inner diameter of the hollow fiber membrane is from 150 $\mu$m to 400 $\mu$m.

(5) The blood treatment material of any of (1) to (4), wherein the material of the hollow fiber membrane is a copolymer of: a hydrophobic polymer selected from the group consisting of polyacrylonitrile, poly(methyl methacrylate), polystyrene, polysulfone, poly(ether-sulfone), polyester, copolymers thereof, and combinations thereof; and a hydrophilic polymer selected from the group consisting of poly(methallylsulfonic acid), poly(methallyl sulfonate), poly(styrenesulfonic acid), poly(styrene sulfonate), sulfonated polysulfone, sulfonated poly(ether-sulfone), copolymers thereof, and combinations thereof and cellulose acetate.

(6) The blood treatment material of any of (1) to (5), for use in adsorbing and removing activated leukocytes and inflammatory cytokines.

(7) A blood purification column comprising the blood treatment material of any of (1) to (6).

Advantageous Effects of Invention

The blood treatment material according to the present invention can highly efficiently adsorb and remove, for example, inflammatory cells and inflammatory cytokines while maintaining water permeability.

Brief Description of Drawing

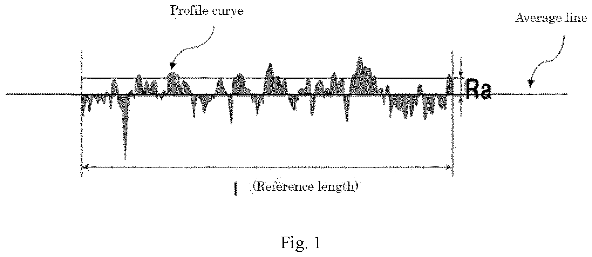

Figure 1 is a diagram explaining a method for determining the arithmetic mean roughness.

Description of Embodiments

The present invention will be described below in detail.

A blood treatment material according to the present invention comprises a hollow fiber membrane having a surface modified with a ligand containing an amino group(s), wherein the arithmetic mean roughness (RaA) of the blood-contacting surface of the hollow fiber membrane in the radial direction calculated by using a laser microscope is from 0.10 $\mu$m to 0.80 $\mu$m.

The term "blood treatment" refers to adsorption and removal of blood components by using an appropriate material.

The "blood components" are components that constitute the blood, and the blood components include, for example, blood cells and humoral factors in the blood.

The term "blood cells" refers to cells in the blood and means, for example, leukocyte components, such as granulocyte, monocyte, neutrophil, and eosinophil, erythrocyte, and platelet.

The term "inflammatory cells" refers to blood cells activated by proteins such as inflammatory cytokines. Specific examples of the inflammatory cells include activated leukocyte, activated platelet, and activated leukocyte-platelet aggregate. In cases where the blood treatment material of the present embodiment is used to treat patients with inflammatory disease or with renal disease complicated by inflammatory disease, an activated leukocyte is a preferred target substance for removal.

The "humoral factors in the blood" are organic materials dissolved in the blood. Specific examples of the organic materials include urea; proteins, such as $\beta$2-microglobulin, cytokines, IgE, and IgG; and polysaccharides, such as lipopolysaccharide (LPS). Among others, proteins such as cytokines and polysaccharides such as LPS are preferred target substances for adsorption and removal in the treatment of patients with inflammatory disease, and urea and β2-microglobulin are preferred target substances for adsorption and removal in the treatment of patients with renal disease. Furthermore, inflammatory cytokines are more preferred target substances for adsorption and removal when the blood treatment material according to the present invention is used to treat patients with inflammatory disease or with renal disease complicated by inflammatory disease.

The term "inflammatory cytokines" refers to a group of proteins that are produced and released extracellularly by various cells, including immune cells, in response to a stimulus such as infection or physical injury, and examples of the inflammatory cytokines include interferon α, interferon β, interferon γ, interleukins 1 through 15, tumor necrosis factor-α, tumor necrosis factor-β, high mobility group box-1, erythropoietin, and monocyte chemoattractant proteins.

The term "adsorption" refers to a state in which a substance is bound to a material and cannot be easily removed, or the state of adsorption equilibrium. Adsorption is not limited to a specific principle but refers to the state of attachment by, for example, electrostatic interaction, hydrophobic interaction, hydrogen bonding, or intermolecular force, such as van der Waals force, or to the state of physical attachment as in cell adhesion or leukocyte phagocytosis.

The "blood treatment material" means a material at least a portion of which comprises a hollow fiber membrane and includes a blood treatment material which comprises only a hollow fiber membrane or which comprises a hollow fiber membrane anchored to or combined with a suitable support material. The anchoring or combination process may precede or follow the formation of a hollow fiber membrane.

A known material used for blood treatment can be employed as a material for the hollow fiber membrane. A preferred material for the hollow fiber membrane is a copolymer of a hydrophobic polymer and a hydrophilic polymer, considering the possibility of swelling of a hollow fiber membrane made of only a water-soluble component and filling the micropores with water in the blood, and the ease of introduction of a ligand. The hydrophobic polymer in this specification is a polymer which is insoluble in water. The phrase "insoluble in water" means that the change in dry weight before and after immersion of the polymer in water is not more than 1%. The change in dry weight refers to the ratio of the dry weight of the remaining solid of the polymer to that of the original polymer before immersion when the polymer is immersed for 1 hour in a volume of water at 37°C that weighs 9 times the dry weight of the polymer and is then removed with tweezers or the like before the polymer is vacuum dried to evaporate the water remaining in the polymer at a temperature of 50°C or lower. Moreover, the hydrophilic polymer in this specification is a polymer which is soluble in water. The phrase "soluble in water" means that the change in the dry weight of the remaining solid is not less than 50% when the polymer is immersed for 1 hour in a volume of water at 37°C that weighs 9 times the dry weight of the polymer and is then removed with tweezers or the like before the polymer is vacuum dried to evaporate the water remaining in the polymer at a temperature of 50°C or lower.

Out of the materials for the hollow fiber membrane, the hydrophobic polymer is, for example, a polymer selected from the group consisting of polyolefins, such as polyethylene and polypropylene, polyacrylates, such as polyacrylonitrile, poly(methyl acrylate), and poly(ethyl acrylate), polymethacrylates, such as poly(methyl methacrylate) and poly(ethyl methacrylate), polystyrene, aromatic poly(ether-sulfone)s, such as polysulfone, and poly(ether-sulfone), aromatic polyetherketones, such as polyetherketone and polyetheretherketone, polyamides, such as polyamide-imide, Nylon-6, and Nylon-6,6, polyurethanes, polyesters, copolymers thereof, and combinations thereof. Because of the ability to copolymerize with a hydrophilic polymer and to readily immobilize a ligand containing an amino group(s), a polymer selected from the group consisting of polyacrylonitrile, poly(methyl methacrylate), polystyrene, polysulfone, poly(ether-sulfone), polyesters, copolymers thereof, and combinations thereof is preferred as the hydrophobic polymer, and a polyacrylonitrile is more preferred.

Out of the materials for the hollow fiber membrane, the hydrophilic polymer is, for example, a polymer selected from the group consisting of sulfonated aromatic poly(ether-sulfone)s, such as poly(methallylsulfonic acid), poly(methallyl sulfonate) (for example, sodium salt, potassium salt, or ammonium salt), poly(styrenesulfonic acid), poly(styrene sulfonate) (for example, sodium salt, potassium salt, or ammonium salt), sulfonated polysulfone, and sulfonated poly(ether-sulfone), polyvinylpyrrolidone, polyethylene glycol, polypropylene glycol, polyvinyl alcohol, copolymers thereof, and combinations thereof, and cellulose, cellulose acetate, carboxymethyl cellulose, and combinations thereof. From the viewpoint of the copolymerization and compatibility with the above hydrophobic polymer, a polymer selected from the group consisting of poly(methallylsulfonic acid), poly(methallyl sulfonate), poly(styrenesulfonic acid), poly(styrene sulfonate), sulfonated polysulfone, sulfonated poly(ether-sulfone), copolymers thereof, combinations thereof, and cellulose acetate is preferred as the hydrophilic polymer, and poly(methallylsulfonic acid) or poly(methallyl sulfonate) is more preferred.

In cases where the material of the hollow fiber membrane is a copolymer of the hydrophobic polymer described above and the hydrophilic polymer described above, the hydrophobic polymer and the hydrophilic polymer may be any combination of a hydrophobic polymer as described above and a hydrophilic polymer as described above. In consideration of the polymerization ability of each monomer and the ease of introduction of a ligand, the material of the hollow fiber membrane is preferably a copolymer of, for example, a hydrophobic polymer and a hydrophilic polymer, the hydrophobic polymer selected from the group consisting of polyacrylonitrile, poly(methyl methacrylate), polystyrene, polysulfone, poly(ether-sulfone), polyester, copolymers thereof, and combinations thereof and the hydrophilic polymer selected from the group consisting of poly(methallylsulfonic acid), poly(methallyl sulfonate), poly(styrenesulfonic acid), poly(styrene sulfonate), sulfonated polysulfone, sulfonated poly(ether-sulfone), copolymers thereof, and combinations thereof and cellulose acetate. More specifically, a copolymer of a polyacrylonitrile and a poly(methallylsulfonic acid) or a salt thereof, a copolymer of a polystyrene and a poly(styrenesulfonic acid) or a salt thereof, a copolymer of a polysulfone and a sulfonated polysulfone, or a copolymer of a poly(ether-sulfone) and sulfonated poly(ether-sulfone) is preferred as the material of the hollow fiber membrane, and a copolymer of a polyacrylonitrile and a poly(methallylsulfonic acid) or a salt thereof is more preferred.

The thickness of the hollow fiber membrane should be set appropriately depending on the usage. For example, the thickness of the hollow fiber membrane is preferably from 20 μm to 80 μm, more preferably from 30 μm to 70 μm, and most preferably from 40 μm to 60 μm, when the hollow fiber membrane is used for extracorporeal circulation.

The inner diameter of the hollow fiber membrane should be set appropriately depending on the thickness and outer diameter of the hollow fiber membrane. For example, the inner diameter of the hollow fiber membrane is preferably from 150 μm to 400 μm and more preferably from 200 μm to 300 μm when the hollow fiber membrane is used for extracorporeal circulation.

The inner diameter of the hollow fiber membrane can be calculated by the following formula 1. In this respect, the thickness refers to the mean of the thickness values measured for randomly selected 16 hollow fiber membranes by the Microwatcher with a lens of 1000 times magnification (for example, VH-Z100; manufactured by Keyence Corporation). Moreover, the outer diameter of the hollow fiber membrane refers to the mean of the outer diameter values measured for randomly selected 16 hollow fiber membranes by laser displacement meter (for example, LS-5040T; manufactured by Keyence Corporation).

$$\text{Inner diameter of hollow fiber membrane (μm)} = \text{Outer diameter of hollow fiber}$$

$$\text{membrane} - 2 \times \text{Thickness of hollow fiber membrane} \qquad \text{Formula 1}$$

A target substance for removal is apparently larger than the actual size due to the influence of Brownian motion or the like. Therefore, when the blood is treated with the hollow fiber membrane, the diameter of the pores on the surface of the hollow fiber membrane should be increased compared to the size of the target substance for removal in order to adsorb the target substance for removal in the micropores. Thus, the diameter of the pores on the surface of the hollow fiber membrane is generally preferably from 0.1 nm to 50 nm and more preferably from 1 nm to 30 nm, though the diameter of the pores depends on the size of a target substance for removal.

The term "water permeability" refers to the amount of water passing through the through holes in the hollow fiber membrane. The presence of pores with a larger pore size or the presence of a larger number of pores increases the water flow channels and consequently increases the water permeability of the hollow fiber membrane. The water permeability of the hollow fiber membrane is preferably from 1.5 L/h/mmHg to 5.0 L/h/mmHg, more preferably from 1.5 L/h/mmHg to 3.0 L/h/mmHg, still more preferably from 1.7 L/h/mmHg to 3.0 L/h/mmHg, and yet more preferably 1.7 L/h/mmHg to 2.5L/h/mmHg. The water permeability can be calculated by the following formula 2.

$$\text{Water permeability (L/h/mmHg)} = \text{Filtration amount (L) / Time (h) / Hydraulic}$$

$$\text{pressure (mmHg)} \qquad \text{Formula 2}$$

The blood-contacting surface of the hollow fiber membrane may be the inner or outer surface of the hollow fiber membrane, and the blood-contacting surface is preferably the inner surface of the hollow fiber membrane because the hollow fiber membrane in this configuration provides a more uniform blood flow channel with less dead space.

The term "arithmetic mean roughness (Ra)" is an index specified in JIS B 0601: 2001 that quantitatively indicates the smoothness of a surface and, in this specification, indicates the roughness of the blood-contacting surface of the hollow fiber membrane in the radial direction. Specifically, a laser microscope (for example, the ultra-deep 3-D shape measuring microscope VK-9710; manufactured by Keyence Corporation) which is a laser confocal optical system capable of two-dimensional scanning and has a line roughness-analyzing function (for example, the shape analysis application VK-H1A1/VK-H2A1; manufactured by Keyence Corporation) is used to acquire images at an objective lens magnification of 50x. For the image acquisition of the hollow fiber membrane, the hollow fiber membrane is first dried and then placed directly on a sample stub to acquire images for analysis of the outer surface or cut into half cylinders to expose the inner surface and then acquire images for analysis of the inner surface. Ten points at different positions in terms of direction and location are randomly selected in an obtained image to extract a line segment in the radial direction of the hollow fiber membrane, and the arithmetic mean roughness can be calculated with reference to the extracted reference length I (in this respect, the line segment is synonymous with the reference length I, which means that the reference length I is 20 $\mu$m when the length of the extracted line segment is 20 $\mu$m). This process is repeated for three images from different fields of view, and the average of the calculated values is taken as the arithmetic mean roughness (Ra) of the hollow fiber membrane. Figure 1 shows an extracted reference length I ($\mu$m), a profile curve, and an average line; an arithmetic mean roughness is a value obtained by averaging the sum of absolute values ($\mu$m) of deviations from the average line to the profile curve along the extracted segment, and the method of calculating the arithmetic mean roughness is expressed by the following formula 3. In this respect, Ra represents an arithmetic mean roughness, and f(x) is a function representing the surface roughness at an arbitrary position x in an image taken by the laser microscope. In this specification, the reference length I for calculating the arithmetic mean roughness (Ra) is 20 $\mu$m.

$$Ra = \frac{1}{\ell} \int_0^\ell \left| f(x) \right| dx$$

Formula 3

For measuring the arithmetic mean roughness (Ra), the hollow fiber membrane should be dried in advance, taking into account a change in shape due to hydration of the surface or a change in wet state due to evaporation of water.

The term "average line" refers to a linear line obtained by applying the least square method to a profile curve, as specified in JIS B 0601: 2001.

The term "profile curve," also known as cross-sectional profile curve, is a curve generated by taking an image of the surface of a material to be studied by using a laser microscope and tracing the surface profile of the material, as shown in Figure 1.

Adequate roughness formed on the blood-contacting surface of the hollow fiber membrane facilitates recognition of the material by blood cells and can increase the specific surface area enough to highly efficiently adsorb and remove humoral factors in the blood. Therefore, the arithmetic mean roughness (Ra) of the blood-contacting surface of the hollow fiber membrane should be from 0.10 $\mu$m to 0.80 $\mu$m and is preferably from 0.20 $\mu$m to 0.70 $\mu$m and more preferably from 0.30 $\mu$m to 0.60 $\mu$m. Any of the preferred lower limit values may arbitrarily be combined with any of the preferred upper limit values.

The "radial direction of the hollow fiber membrane" means the direction perpendicular to the longitudinal direction of the hollow fiber membrane, which is the moving direction of the hollow fiber membrane discharged during the spinning process (discharge method), and can also be referred to as the circumferential direction.

The "ligand" means a compound that modifies at least the blood-contacting surface of the hollow fiber membrane. In cases where the inner surface of the hollow fiber membrane is a blood-contacting surface, the inner surface is modified with the ligand. In cases where the outer surface of the hollow fiber membrane is a blood-contacting surface, the outer surface is modified with the ligand. The non-blood-contacting surface may or may not be modified with the ligand. It is preferred that the ligand be distributed over the entire modified surface. The ligand is not specifically limited in terms of chemical structure, provided that the ligand contains an amino group(s).

Examples of the "amino group" include an amino group derived from a primary amine, such as methylamine, ethylamine, propylamine, butylamine, pentylamine, hexylamine, heptylamine, octylamine, or dodecylamine; an amino group derived from a secondary amine, such as methylhexylamine, diphenylmethylamine, or dimethylamine; an amino group derived from an amine having an unsaturated alkyl chain, such as allylamine; an amino group derived from a tertiary amine, such as trimethylamine, triethylamine, dimethylethylamine, phenyldimethylamine, dimethylhexylamine; an amino group derived from a amine having an aromatic ring, such as 1-(3-aminopropyl)imidazole, pyridine-2-amine, or 3-sulfoaniline; or an amino group derived from a compound containing two or more amino groups linked by, for example, an alkyl chain, an aromatic compound, a heterocyclic compound, or a carbocyclic compound (hereinafter referred to as "polyamine"), such as tris(2-aminoethyl)amine, ethylenediamine, diethylenetriamine, triethylenetetramine, tetraethylenepentamine, pentaethylenehexamine, hexaethyleneheptamine, heptaethyleneoctamine, octaethylenenonamine, dipropylenetriamine, polyethyleneimine, N-methyl-2,2'-diaminodiethylamine, N-acetylethylenediamine, 1,2-bis(2-aminoethoxyethane). An amino group derived from ethylenediamine, diethylenetriamine, triethylenetetramine, tetraethylenepentamine, pentaethylenehexamine, hexaethyleneheptamine, heptaethyleneoctamine, octaethylenenonamine, dipropylenetriamine, polyethyleneimine, N-methyl-2,2'-diaminodiethylamine, N-acetylethylenediamine, or 1,2-bis(2-aminoethoxyethane) is more preferred; an amino group derived from ethylenediamine, diethylenetriamine, triethylenetetramine, tetraethylenepentamine, polyethyleneimine, or polyethyleneimine is still more preferred. The polyamines described above may be linear, branched, or cyclic. Moreover, hydrogen atoms attached to the basic nitrogen atoms in the polyamines described above may be replaced by a substituent(s), for example an unsaturated alkyl group(s), such

as $C_{1-10}$ alkyl group, vinyl group, or allyl group, an aryl group(s), such as phenyl group, naphthyl group, or anthracyl group, or a heteroaryl group(s), such as imidazolyl group, pyridyl group, or piperidyl group. The replacement is not specifically limited in terms of the number and location of substituents.

In the hollow fiber membrane, the surface should be modified with a ligand containing an amino group(s) (for example, an amino group derived from a polyamine). In examples of the way of modifying the surface, the surface of the hollow fiber membrane may be modified with the ligand by electrostatic interaction between the membrane surface and the ligand (for example, electrostatic interaction between an anionic functional group such as sulfonate group, and a cationic functional group, such as polyamine) or by direct binding of a predetermined functional group on the surface of the hollow fiber membrane to the ligand (via covalent bonding, hydrogen bonding, or the like) or by binding of a predetermined functional group on the surface of the hollow fiber membrane to the ligand via a spacer derived from a reactive functional group. Examples of the spacer include sulfonate, amide, carboxyl, sulfonamide, ureido, and ether groups. In cases where the hollow fiber membrane contains an anionic functional group (for example, sulfonate group) as a constituent, the surface modification is preferably performed by electrostatic interaction because of the simplicity.

The amino group content is not specifically limited, and an amino group content of not less than 0.05 mmol per 1 g dry weight of the hollow fiber membrane is preferred to achieve satisfactory adsorption performance against charged organic compounds such as blood components, and an amino group content of not more than 1.00 mmol per 1 g dry weight of the hollow fiber membrane is preferred in consideration of the effect on the blood pH. That is, the amino group content is preferably in the range of 0.05 mmol to 1.00 mmol, more preferably 0.10 mmol to 0.50 mmol, still more preferably 0.10 mmol to 0.30 mmol, per 1 g dry weight of the hollow fiber membrane. Any of the preferred lower limit values may be combined with any of the preferred upper limit values.

The amino group content can be measured by an acid-base titration method using hydrochloric acid or an aqueous solution of sodium hydroxide.

In the case of a hollow fiber membrane composed of an acrylonitrile-sodium methallyl sulfonate copolymer, the hollow fiber membrane can be produced by, for example but not limited to, the following method.

Acrylonitrile and sodium methallyl sulfonate are added to DMSO and polymerized to form a polymer solution with a viscosity $[\eta]$ of 3.2, and the resulting solution is further diluted to obtain a spinning solution containing the polymer at a total concentration of 13.0% by weight. Then, a spinneret with an inner diameter of 0.25 mm and a slit width of 0.1 mm for a sheath-core-type hollow fiber membrane is used to extrude the spinning solution at a rate of 1.4 ml/min from the sheath hole of the spinneret and to inject a coagulant solution, which is an aqueous solution of DMSO at a concentration of 80% by weight, from the core hole of the spinneret. The temperature of the spinneret is 50°C, and the extruded filament is first allowed to travel 80 mm in the air (at room temperature) and then led to a coagulation bath consisting of water at 30°C for coagulation, and the resulting filament is washed in water at 35°C and reeled to obtain a hollow fiber membrane.

For the addition ratio of acrylonitrile and sodium methallyl sulfonate in the production of the spinning solution described above, the amount of acrylonitrile added is preferably in the range of 60 to 95% by mole from the viewpoint of spinnability and fiber strength, and the amount of sodium methallyl sulfonate added is preferably in the range of 5 to 40% by mole for surface modification with a ligand to sufficiently adsorb and remove a target substance for removal.

In cases where the ligand is a polyamine, a method of modifying the hollow fiber membrane produced by the above method with the ligand is, for example but not limited to, a method comprising immersing the hollow fiber membrane in a solution of the polyamine or a method comprising supplying a solution of the polyamine to the inside of the hollow fiber membrane.

A solvent for dissolving the polyamine is, for example, water, an aqueous solution containing a salt (for example, phosphate buffer, borate buffer, ammonium chloride buffer, aqueous solution of sodium hydroxide, brine, aqueous solution of sodium carbonate, or aqueous solution of sodium bicarbonate), or a mixed solvent thereof, and water is a preferred solvent.

Preferably, the solution prepared by dissolving the polyamine has a pH of 7 to 12.

Preferably, the concentration of the polyamine in the solution prepared by dissolving the polyamine is from 0.04 g/L to 20 g/L.

The immersion temperature at which the hollow fiber membrane is immersed preferably ranges from 10 to 80°C and more preferably from 20 to 60°C.

The immersion time during which the hollow fiber membrane is immersed preferably ranges from 30 minutes to 24 hours and more preferably 1 hour to 8 hours.

From the viewpoint of maintaining the water permeability, which is one of the characteristics of the hollow fiber membrane, it is common that the hollow fiber membrane is directly packed into a column after spinning and is then surface-treated by feeding a surface treatment solution prepared with water into the hollow fiber membrane, if necessary, to modify the surface of the membrane. Therefore, the surface of the hollow fiber membrane produced by the above method is relatively smooth and not rough, whether modified with a ligand or not. It should be noted that the blood-contacting surface of the hollow fiber membrane produced by the above production method is confirmed to be almost free of roughness in Comparative Examples below.

The inventors have found that the surface property of the hollow fiber membrane can be modified by using an organic solvent to roughen the surface of the hollow fiber membrane while maintaining the water permeability of the hollow fiber membrane. A method for roughening the blood-contacting surface of the hollow fiber membrane is, for example but not limited to, a method comprising immersing the hollow fiber membrane in a solvent described below or a method comprising supplying a solvent described below to the inside of the hollow fiber membrane.

Examples of the solvent include N,N-dimethylformamide, N,N-dimethylacetamide, diethyl ether, dioxane, tetrahydrofuran, acetonitrile, acetone, dimethyl sulfoxide, and mixed solvents of water and any of these solvents, as well as toluene, xylene, nitrobenzene, chloroform, dichloromethane, carbon tetrachloride, hexane, and heptane. N,N-dimethylformamide, N,N-dimethylacetamide, dioxane, tetrahydrofuran, acetonitrile, acetone, dimethyl sulfoxide, or a mixture of water and any of these solvents is preferred; dimethyl sulfoxide or a mixed solvent of dimethyl sulfoxide and water is more preferred; a mixed solvent of dimethyl sulfoxide and water is still more preferred. Any one of these solvents may be used alone, or any two or more of these solvents may be combined to form a mixed solvent.

In cases where a mixed solvent of, for example, dimethyl sulfoxide and water is used as the mixed solvent, the concentration of dimethyl sulfoxide is preferably in the range of 10 to 50% by weight. The same is true when using a mixed solvent of another solvent and water.

The immersion temperature at which the hollow fiber membrane is immersed preferably ranges from 10 to 80°C and more preferably from 20 to 60°C.

When the hollow fiber membrane is immersed, too long immersion may cause excessive dissolution of the hollow fiber membrane, depending on the material of the hollow fiber membrane. On the contrary, too short immersion may cause failure to adequately roughen the surface of the membrane. Therefore, the immersion time is generally preferably between 30 minutes and 5 hours, more preferably between 1 hour and 4 hours, and still more preferably between 1 hour and 3 hours.

When a solvent is supplied to the inside of the hollow fiber membrane, a flow rate that is too fast may cause excessive dissolution of the hollow fiber membrane, depending on the material of the hollow fiber membrane. Therefore, the flow rate is preferably from 0.1 to 2.0 mL/min and more preferably from 0.3 to 1.5 mL/min. The same is true when a solvent is supplied outside the hollow fiber membrane.

When a solvent is supplied to the inside of the hollow fiber membrane, supplying the solvent for too long period of time may cause excessive dissolution of the hollow fiber membrane, depending on the material of the hollow fiber membrane. Therefore, the duration of the solvent supply is generally preferably ranges between 30 minutes and 5 hours, more preferably between 1 hour and 4 hours, and still more preferably between 1 hour and 3 hours. The same is true when a solvent is supplied outside the hollow fiber membrane.

When a solvent is supplied to the inside of the hollow fiber membrane, the temperature of the solvent preferably ranges from 10 to 80°C and more preferably from 20 to 60°C. The same is true when a solvent is supplied outside the hollow fiber membrane.

The blood treatment material of the present embodiment can be used as a support packed in a blood purification column. In cases where a blood treatment material is packed in a blood purification column, the blood treatment material may be a hollow fiber membrane bundle comprising multiple hollow fiber membranes. Such a hollow fiber membrane bundle is suitable for use as a support for adsorption and removal of activated leukocytes and/or inflammatory cytokines, especially in cases of extracorporeal circulation for treatment of patients with inflammatory disease. In cases where a blood purification column comprising the blood treatment material is used as a column of an extracorporeal circulation system for the blood purification therapy, the blood drawn out of the body may be sent directly to the column, or the column may be used in combination with a plasma separation membrane or the like.

The term "inflammatory disease" represents all diseases which cause an inflammatory response in the body; examples of inflammatory diseases include systemic lupus erythematosus, malignant rheumatoid arthritis, multiple sclerosis, ulcerative colitis, Crohn's disease, drug-induced hepatitis, alcoholic hepatitis, hepatitis A, hepatitis B, hepatitis C, hepatitis D, or hepatitis E, sepsis (for example, gram-negative sepsis, gram-positive sepsis, culture-negative sepsis, and fungal sepsis), influenza, acute respiratory distress syndrome (ARDS), acute lung injury (ALI), pancreatitis, idiopathic pulmonary fibrosis (IPF), inflammatory bowel disease (for example, ulcerative colitis, Crohn's disease), blood product transfusion, organ transplantation, reperfusion injury after organ transplantation, cholecystitis, cholangitis, and neonatal blood type incompatibility. Diseases that are caused by a causative substance(s) released into the blood and for which the therapeutic effect potential of the blood purification is very promising include, among those inflammatory diseases, drug-induced hepatitis, alcoholic hepatitis, hepatitis A, hepatitis B, hepatitis C, hepatitis D, or hepatitis E, sepsis (for example, gram-negative sepsis, gram-positive sepsis, culture-negative sepsis, and fungal sepsis), influenza, acute respiratory distress syndrome, acute lung injury, pancreatitis, and idiopathic pulmonary fibrosis.

For example, a method of evaluating the blood purification performance of a blood treatment material is a method comprising determining the activated leukocyte removal ratio. For example, the method of determining the activated leukocyte removal ratio is a method comprising packing a container having an inlet and an outlet with a blood treatment material, supplying a fluid containing activated leukocytes to the container, and calculating the ratio of removed activated leukocytes from the change between the activated leukocyte concentration at the inlet and that at the outlet.

Since activated leukocytes are cells and the degree of leukocyte activation varies from person to person, a ratio of activated leukocyte removal of not less than 6% indicates significant removal of activated leukocytes. However, the activated leukocyte removal ratio is preferably not more than 80%, because the blood supplied to the inside of a hollow fiber membrane may cause excessive adsorption of activated leukocytes by the hollow fiber membrane, leading to clogging of the hollow fiber membrane and an increase in circulatory pressure.

Another method for evaluating the blood purification performance of a blood treatment material is a method comprising determining the interleukin-8 (hereinafter referred to as IL-8) adsorption ratio. IL-8 is an inflammatory cytokine found in blood components and is known to be significantly elevated in the blood components of patients with inflammatory disease, especially with bronchiolitis or diseases caused by viral infections. Therefore, IL-8 is suitable as a blood component for evaluating the blood purification performance. A higher IL-8 adsorption ratio of a blood treatment material indicates a higher blood purification performance of the blood treatment material.

In addition, the blood purification column of the present invention is characterized by comprising the blood treatment material described above.

The blood purification column of the present embodiment can be used for purifying or removing a blood component of interest from a fluid containing blood components and the like, for example for separating a particular blood component. The blood purification column of the present embodiment is preferably used for adsorbing and removing, among blood components, blood cells and humoral factors in the blood; the blood purification column of the present embodiment is particularly suitable for use as a blood purification column for adsorbing and removing activated leukocytes and/or inflammatory cytokines, among others.

A container for the blood purification column may have any shape, provided that the container has an inlet and an outlet for a fluid containing blood components and the like, an inlet and an outlet for a dialysis solution, and a housing, and a blood treatment material may be packed in the housing. In one embodiment, a hollow fiber membrane is cut to a required length, and a required number of cut pieces of the hollow fiber membrane are bundled and then placed in a cylindrical housing. Both ends of the housing are then temporarily capped, and a potting agent is applied to both ends of the hollow fiber membranes. After the potting agent has set, the tips of the hollow fiber membranes are cut off at both ends to expose the open ends of the hollow fiber membranes. Headers are attached to both ends of the housing, and plugs are inserted into the headers and nozzles of the housing to obtain a hollow fiber membrane-packed column. The packing ratio of the hollow fiber membranes (the volume ratio of the hollow fiber membranes to the housing) is preferably not more than 80%, still more preferably not more than 70%, from the viewpoint of a uniform flow of a sufficient amount of the dialysis solution. Additionally, the packing ratio of the hollow fiber membranes (the volume ratio of the hollow fiber membranes to the housing) is preferably not less than 20%, still more preferably not less than 30%, to maintain the capacity of one column to adsorb and remove activated leukocytes and/or inflammatory cytokines.

The blood purification column may be packed with a blood treatment material alone or in combination with another hollow fiber membrane and/or with various types of fillers. Examples of the fillers include fibrous materials in sheet form such as knitted, woven, and nonwoven fabrics, membranes, particles, and hydrogels. In this respect, the blood treatment material filled in the column is preferably a hollow fiber membrane bundle composed of multiple hollow fiber membranes.

The blood purification column can be used for, for example, the aforementioned treatment of patients with inflammatory disease or with renal disease or patients with renal disease complicated by inflammatory disease. Renal diseases include chronic renal failure and acute renal failure.

In the treatment of patients with renal failure, blood purification therapies using blood purification columns are widely used for the purpose of removing uremic toxins and waste products in the blood, wherein a dialysis membrane or an ultrafiltration membrane is used as a separation material in the blood purification column, and wherein a naturally occurring polymer, such as cellulose, or a synthetic polymer, such as polysulfone, poly(methyl methacrylate), or polyacrylonitrile, is used for the dialysis membrane or the ultrafiltration membrane. In particular, blood purification columns using hollow fiber membranes as the separation material are highly important in the field of dialyzer technology because of advantages such as a reduced volume of extracorporeally circulated blood and an high efficiency in removing substances from the blood.

The blood purification column of the present embodiment is expected to improve the unstable hemodynamics when used for the treatment of patients suffering from both an inflammatory disease as described above, such as acute lung injury, and a renal disease, such as acute renal failure. Specifically, the blood purification column of the present embodiment can be expected to adsorb and remove excessive inflammatory cytokines and/or inflammatory cells in the blood in the treatment of inflammatory diseases and to remove an excess amount of water from the blood of the patients, which contains too much water due to renal failure or the like, in the treatment of renal diseases.

Examples

The blood treatment material of the present invention will be specifically described by examples below, but the present invention is not limited to these examples.

(Production of Blood Treatment Material 1)

A "sepXiris®" (Baxter Ltd., medical device approval number: 22500BZX00401000) was cut with a pipe cutter to recover a hollow fiber membrane made of a copolymer of acrylonitrile and sodium methallyl sulfonate and coated with polyethyleneimine on the surface was recovered from, and the recovered hollow fiber membrane (inner diameter: 240 $\mu$m, film thickness: 50 $\mu$m) was designated as the blood treatment material 1. The blood treatment material 1 was analyzed to determine the amino group content of 1 g dry weight of the blood treatment material 1 and the arithmetic mean surface roughness (Ra) of the blood treatment material 1.

Measurement of the amino group content of the blood treatment material 1:

The amino group content of the blood treatment material 1 was determined by acid-base back-titration of the blood treatment material 1. Into a 200-cm$^3$ eggplant flask, 1.5 g of the blood treatment material 1 was added, and the flask was left to stand in a dryer at 80°C for 48 hours under normal pressure to obtain a dried blood treatment material 1. Then, 1.0 g of the blood treatment material 1 described above and 50 cm$^3$ of a 6 M aqueous solution of sodium hydroxide were added to a polypropylene container and stirred for 30 minutes, and the blood treatment material 1 was filtered out by using filter paper. Then, the blood treatment material 1 described above was placed in 50 cm$^3$ of ion-exchanged water and stirred for 30 minutes, and the blood treatment material 1 was filtered out by using filter paper. The process comprising placing the blood treatment material 1 described above in ion-exchanged water and washing and filtering the blood treatment material 1 was repeated until the filtrate obtained by filtering the added ion-exchanged water reached pH 7, to obtain a desalted blood treatment material 1. The desalted blood treatment material 1 was left to stand under vacuum in a vacuum dryer at 30°C for 8 hours. Subsequently, 1.0 g of the blood treatment material 1 described above and 30 cm$^3$ of 0.1M hydrochloric acid were added to a polypropylene container and stirred for 10 minutes. After stirring, 5 cm$^3$ of the solution alone was withdrawn and transferred to a polypropylene container. Then, 0.1 cm$^3$ of a 0.1 M aqueous solution of sodium hydroxide was added dropwise to the withdrawn aliquot of the solution. After the dropwise addition was completed, the mixture was stirred for 10 minutes to measure the pH of the resulting solution. Each time the volume of the 0.1 M aqueous sodium hydroxide solution was added dropwise, the mixture was stirred for 10 minutes to measure the pH of the resulting solution, and the same operation was repeated 100 times. The amount of 0.1 M aqueous sodium hydroxide solution added to bring the pH of the solution above 8.5 was referred to as the titer of 1 g of the blood treatment material 1. The amino group content of 1 g of the blood treatment material 1 was calculated by using the titer of 1 g of the blood treatment material 1 and the following formula 4. The result is shown in Table 1.

$$\text{Amino group content of 1 g dry weight of the blood treatment material 1 (mmol/g)} =$$

$$\{\text{Volume of the 0.1 M hydrochloric acid added (30 cm}^3) / \text{Volume of the withdrawn}$$

$$\text{hydrochloric acid (5 cm}^3)\} \times \text{Titer of 1 g of the blood treatment material 1 (cm}^3/\text{g}) \times$$

$$\text{Concentration of the aqueous sodium hydroxide solution (0.1 mol/L)}$$

$$\text{Formula 4}$$

Measurement of the arithmetic mean roughness (Ra) of the inner surface of the blood treatment material 1 in the radial direction:

The blood treatment material 1 was cut into a piece of 5 cm in length, and the piece of the blood treatment material 1 was further cut into half cylinders to expose the inner surface and dried under vacuum at 25°C for 16 hours. The image of the inner surface of the dried blood treatment material 1 was taken at an objective lens magnification of 50x by using a laser microscope (ultra-deep 3-D shape measuring microscope VK-9710; manufactured by Keyence Corporation). Ten points at different positions in terms of direction and location were randomly selected from the profile curve of one hollow fiber membrane in the obtained image, to extract the line segment in the radial direction of the hollow fiber membrane, where the reference length l was 20 μm. The analysis software included with the VK-9710 was used for the analysis in the line roughness mode to measure the arithmetic mean roughness (Ra) of the inner surface in the radial direction (in accordance with JIS B 0601: 2001). The process described above was repeated for three images from different fields of view, and the average of the calculated values was taken as the arithmetic mean roughness (Ra) of the inner surface of the blood treatment material 1 in the radial direction. The result is shown in Table 1.

Measurement of the water permeability of the blood treatment material 1:

A hollow fiber membrane module was produced by bundling 124 pieces of the blood treatment material 1, inserting the resulting bundle into a housing with a diameter of about 5 mm and a length of 10 cm, potting both ends of the bundle of hollow fiber membranes with an epoxy resin-based chemical reaction-type adhesive "Quick Mender®" manufactured by Konishi Co., Ltd., and cutting off the tips of the hollow fiber membranes at both ends to expose the open ends. Then, the inside of the hollow fiber membranes in the module was filled with water, and a hydraulic pressure of 100 mmHg was applied to the inside of the hollow fiber membranes for 15 minutes to measure the volume of filtrate flowing outside the hollow fiber membranes in a unit time period, and the water permeability was calculated by the following formula 2.

$$\text{Water permeability (L/h/mmHg)} = \text{Filtration amount (L)} / \text{Time (h)} / \text{Hydraulic pressure (mmHg)} \qquad \text{Formula 2}$$

(Production of Blood Treatment Material 2)

A hollow fiber membrane module was produced by bundling 124 pieces of the blood treatment material 1 described above, inserting the resulting bundle into a housing with a diameter of about 5 mm and a length of 10 cm, potting both ends of the bundle of hollow fiber membranes with an epoxy resin-based chemical reaction-type adhesive "Quick Mender®" manufactured by Konishi Co., Ltd., and cutting off the tips of the hollow fiber membranes at both ends to expose the open ends. Then, the inside of the hollow fiber membranes in the module was supplied with an aqueous solution of DMSO at a concentration of 50% by weight and at a temperature of 30°C at a flow rate of 0.63 mL/min for 1 hour and with water at a flow rate of 0.63 mL/min for 1 hour to obtain the blood treatment material 2.

Measurement of the amino group content of the blood treatment material 2:

The amino group content of the blood treatment material 2 was measured in the same manner as for the blood treatment material 1. The result is shown in Table 1.

Measurement of the arithmetic mean roughness (Ra) of the inner surface of the blood treatment material 2 in the radial direction:

The arithmetic mean roughness (Ra) of the inner surface of the blood treatment material 2 in the radial direction was measured in the same manner as for the blood treatment material 1. The result is shown in Table 1.

Measurement of the water permeability of the blood treatment material 2:

The water permeability of the blood treatment material 2 was measured in the same manner as for the blood treatment material 1. The result is shown in Table 1.

(Production of Blood Treatment Material 3)

The blood treatment material 3 was produced in the same manner as the production method of the blood treatment material 2, except that the time period of supplying the aqueous solution of DMSO at a concentration of 50% by weight was changed to 2 hours.

Measurement of the amino group content of the blood treatment material 3:

The amino group content of the blood treatment material 3 was measured in the same manner as for the blood treatment material 1. The result is shown in Table 1.

Measurement of the arithmetic mean roughness (Ra) of the inner surface of the blood treatment material 3 in the radial direction:

The arithmetic mean roughness (Ra) of the inner surface of the blood treatment material 3 in the radial direction was measured in the same manner as for the blood treatment material 1. The result is shown in Table 1.

Measurement of the water permeability of the blood treatment material 3:

The water permeability of the blood treatment material 3 was measured in the same manner as for the blood treatment material 1. The result is shown in Table 1.

(Production of Blood Treatment Material 4)

The blood treatment material 4 was produced in the same manner as the production method of the blood treatment material 2, except that the time period of supplying the aqueous solution of DMSO at a concentration of 50% by weight was changed to 3 hours.

Measurement of the amino group content of the blood treatment material 4:

The amino group content of the blood treatment material 4 was measured in the same manner as for the blood treatment material 1. The result is shown in Table 1.

Measurement of the arithmetic mean roughness (Ra) of the inner surface of the blood treatment material 4 in the radial direction:

The arithmetic mean roughness (Ra) of the inner surface of the blood treatment material 4 in the radial direction was measured in the same manner as for the blood treatment material 1. The result is shown in Table 1.

Measurement of the water permeability of the blood treatment material 4:

The water permeability of the blood treatment material 4 was measured in the same manner as for the blood treatment material 1. The result is shown in Table 1.

(Production of Blood Treatment Material 5)

The blood treatment material 5 was produced in the same manner as the production method of the blood treatment material 2, except that the concentration of DMSO in the aqueous solution of DMSO was changed to 10% by weight.

Measurement of the amino group content of the blood treatment material 5:

The amino group content of the blood treatment material 5 was measured in the same manner as for the blood treatment material 1. The result is shown in Table 1.

Measurement of the arithmetic mean roughness (Ra) of the inner surface of the blood treatment material 5 in the radial direction:

The arithmetic mean roughness (Ra) of the inner surface of the blood treatment material 5 in the radial direction was measured in the same manner as for the blood treatment material 1. The result is shown in Table 1.

Measurement of the water permeability of the blood treatment material 5:

The water permeability of the blood treatment material 5 was measured in the same manner as for the blood treatment material 1. The result is shown in Table 1.

(Production of Blood Treatment Material 6)

The blood treatment material 6 was produced in the same manner as the production method of the blood treatment material 2, except that the time period of supplying the aqueous solution of DMSO at a concentration of 50% by weight was changed to 6 hours.

Measurement of the amino group content of the blood treatment material 6:

The amino group content of the blood treatment material 6 was measured in the same manner as for the blood treatment material 1. The result is shown in Table 1.

Measurement of the arithmetic mean roughness (Ra) of the inner surface of the blood treatment material 6 in the radial direction:

The arithmetic mean roughness (Ra) of the inner surface of the blood treatment material 6 in the radial direction was measured in the same manner as for the blood treatment material 1. The result is shown in Table 1.

Measurement of the water permeability of the blood treatment material 6:

The water permeability of the blood treatment material 6 was measured in the same manner as for the blood treatment material 1. The result is shown in Table 1.

(Example 1)

Measurement of the activated leukocyte removal ratio by the blood treatment material 2:

A hollow fiber membrane module was produced by bundling 124 pieces of the blood treatment material 2, inserting the resulting bundle into a housing with a diameter of about 5 mm and a length of 10 cm, potting both ends of the bundle of hollow fiber membranes with an epoxy resin-based chemical reaction-type adhesive "Quick Mender®" manufactured by Konishi Co., Ltd., and cutting off the tips of the hollow fiber membranes at both ends to expose the open ends. Then, the blood from a healthy human volunteer supplemented with LPS to a concentration of 70 EU/mL was activated at 37°C with stirring at 65 rpm for 30 minutes, and the resulting activated blood was supplied to the inside of the hollow fiber membranes in the module at a flow rate of 0.63 mL/min, and blood samples were collected at the inlet and outlet of the module. The time at which the blood entered the module was taken as 0 minutes, and an aliquot of the blood that circulated through the module and reached the outlet of the module 6.5 minutes later was taken as the outlet sample. The collected samples were analyzed with a multispecies automated hematology analyzer to determine the activated leukocyte removal ratio by the blood treatment material 2 by using the following formula 5. The result is shown in Table 2.

$$\text{Ratio of activated leukocyte removal (\%)} = (\text{Concentration of activated leukocytes in}$$
$$\text{the blood after the blood circulation test } (10^2 \text{ cells/}\mu\text{L})) / (\text{Concentration of activated}$$
$$\text{leukocytes in the blood before the blood circulation test } (10^2 \text{ cells/}\mu\text{L}))$$

$$\text{Formula 5}$$

Measurement of the IL-8 adsorption ratio of the blood treatment material 2:

In order to determine the IL-8 adsorption performance of the blood treatment material 2, the blood treatment material 2 was immersed in a liquid containing IL-8 for a predetermined period of time and then taken out to measure the IL-8 adsorption ratio from the difference in the amount of IL-8 before and after the immersion. The measurement method is described below.

A portion of the blood treatment material 2 equal to 50 cm in length was sampled and placed in a polypropylene container. A solution of fetal bovine serum (hereinafter referred to as FBS) prepared to contain IL-8 at a concentration of 2000 pg/mL was added to the container at a ratio of 88 mL per 1 cm³ of the blood treatment material 2 and was agitated by rotation for 1 hour in an incubator at 37°C, and the concentration of IL-8 in the FBS solution was then measured by enzyme-linked immunosorbent assay (ELISA). The IL-8 adsorption ratio was calculated from the concentration of IL-8 before and after rotary agitation according to the following formula 6. The result is shown in Table 2.

$$\text{IL-8 adsorption ratio (\%)} = \{\text{Concentration of IL-8 before rotary agitation (pg/mL)} -$$

$$\text{Concentration of IL-8 after rotary agitation (pg/mL)}\} / \text{Concentration of IL-8 before}$$

$$\text{rotary agitation (pg/mL)} \times 100 \qquad\qquad \text{Formula 6}$$

(Example 2)

The activated leukocyte removal ratio and the IL-8 adsorption ratio were determined by the same measurements as in Example 1 using the blood treatment material 3. The result is shown in Table 2.

(Example 3)

The activated leukocyte removal ratio and the IL-8 adsorption ratio were determined by the same measurements as in Example 1 using the blood treatment material 4. The result is shown in Table 2.

(Example 4)

The activated leukocyte removal ratio and the IL-8 adsorption ratio were determined by the same measurements as in Example 1 using the blood treatment material 5. The result is shown in Table 2.

(Comparative Example 1)

The activated leukocyte removal ratio and the IL-8 adsorption ratio were determined by the same measurements as in Example 1 using the blood treatment material 1. The result is shown in Table 2.

(Comparative Example 2)

The activated leukocyte removal ratio and the IL-8 adsorption ratio were determined by the same measurements as in Example 1 using the blood treatment material 6. The result is shown in Table 2.

Table 1

| Examples | Amino Group Content (mmol/g) | Arithmetic Mean Roughness (Ra) ($\mu$m) | Water Permeability (L/h/mmHg) |
|---|---|---|---|
| Blood treatment material 1 | 0.13 | 0.04 | 2.3 |
| Blood treatment material 2 | 0.12 | 0.35 | 2.0 |
| Blood treatment material 3 | 0.10 | 0.50 | 2.0 |
| Blood treatment material 4 | 0.09 | 0.78 | 1.7 |
| Blood treatment material 5 | 0.12 | 0.11 | 2.4 |
| Blood treatment material 6 | 0.08 | 0.93 | 1.4 |

In Table 1, the contents of amino groups present on the surface of the hollow fiber membranes are indicated in the "Amino Group Content" column, and the arithmetic mean roughness (Ra) values of the inner surfaces of the hollow fiber membranes are indicated in the radial direction are indicated in the "Arithmetic Mean Roughness (Ra)" column.

Table 2

| Examples | IL-8 Adsorption (%) | Activated Leukocyte Removal (%) |
|---|---|---|
| Example 1 | 80 | 29 |
| Example 2 | 78 | 40 |
| Example 3 | 54 | 41 |
| Example 4 | 85 | 10 |
| Comparative Example 1 | 89 | 3 |
| Comparative Example 2 | 24 | 38 |

In Table 2, the ratios of IL-8 adsorbed and removed by the blood treatment materials are indicated in the "IL-8 Adsorption Ratio" column, and the ratios of activated leukocytes adsorbed and removed by the blood treatment materials are indicated in the "Activated Leukocyte Removal Ratio" column.

These results indicate that the blood treatment materials of the present application can adsorb and remove activated leukocytes and IL-8 more efficiently than the blood treatment materials comprising hollow fiber membranes having an inner surface, which is the blood-contacting surface, with an arithmetic mean roughness (Ra) of less than 0.10 $\mu$m or more than 0.80 $\mu$m in the radial direction.

Industrial Applicability

The blood treatment materials of the present invention have both water permeability and the ability to highly efficiently adsorb and remove, for example, activated leukocytes and inflammatory cytokines, and can therefore be used as an adsorptive support for extracorporeal circulation.

**Claims**

1. A blood treatment material comprising a hollow fiber membrane having a surface modified with a ligand containing an amino group(s),
   wherein the arithmetic mean roughness (RaA) of the blood-contacting surface of the hollow fiber membrane in the radial direction calculated by using a laser microscope is from 0.10 $\mu$m to 0.80 $\mu$m.

2. The blood treatment material of claim 1, wherein the arithmetic mean roughness (RaA) is from 0.30 $\mu$m to 0.60 $\mu$m.

3. The blood treatment material of claim 1 or 2, wherein the blood-contacting surface is the inner surface of the hollow fiber membrane.

4. The blood treatment material of any one of claims 1 to 3, wherein the inner diameter of the hollow fiber membrane is from 150 $\mu$m to 400 $\mu$m.

5. The blood treatment material of any one of claims 1 to 4, wherein the material of the hollow fiber membrane is a copolymer of:
   a hydrophobic polymer selected from the group consisting of polyacrylonitrile, poly(methyl methacrylate), polystyrene, polysulfone, poly(ether-sulfone), polyester, copolymers thereof, and combinations thereof; and a hydrophilic polymer selected from the group consisting of poly(methallylsulfonic acid), poly(methallyl sulfonate), poly(styrenesulfonic acid), poly(styrene sulfonate), sulfonated polysulfone, sulfonated poly(ether-sulfone), copolymers thereof, and combinations thereof and cellulose acetate.

6. The blood treatment material of any one of claims 1 to 5, for use in adsorbing and removing activated leukocytes and inflammatory cytokines.

7. A blood purification column comprising the blood treatment material of any one of claims 1 to 6.

Fig. 1

## INTERNATIONAL SEARCH REPORT

International application No.

**PCT/JP2022/017891**

### A. CLASSIFICATION OF SUBJECT MATTER

*A61M 1/18*(2006.01)i; *A61M 1/36*(2006.01)i
FI:　A61M1/18 500; A61M1/36 165

According to International Patent Classification (IPC) or to both national classification and IPC

### B. FIELDS SEARCHED

Minimum documentation searched (classification system followed by classification symbols)

　　A61M1/18; A61M1/36

Documentation searched other than minimum documentation to the extent that such documents are included in the fields searched

　　Published examined utility model applications of Japan 1922-1996
　　Published unexamined utility model applications of Japan 1971-2022
　　Registered utility model specifications of Japan 1996-2022
　　Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2021/066152 A1 (TORAY INDUSTRIES, INC.) 08 April 2021 (2021-04-08) paragraphs [0013], [0014], [0019]-[0031],[0040]-[0056], [0075], [0129]-0131] | 1-7 |
| A | JP 2011-145 A (NIKKISO CO., LTD.) 06 January 2011 (2011-01-06) paragraph [0011] | 1 |

☐ Further documents are listed in the continuation of Box C.　　☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **10 June 2022** | **21 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2022/017891**

| Patent document cited in search report | Publication date (day/month/year) | Patent family member(s) | Publication date (day/month/year) |
|---|---|---|---|
| WO 2021/066152 A1 | 08 April 2021 | CN 114423470 A | |
| JP 2011-145 A | 06 January 2011 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4224621 B
- JP 4579916 B
- JP 6378090 B
- JP 5298719 B